# EUROPÄISCHE PATENTSCHRIFT

(11) **EP 3 906 009 B1**
(45) Veröffentlichungstag und Bekanntmachung des Hinweises auf die Patenterteilung: **18.12.2024**
(21) Anmeldenummer: 20706181.3
(22) Anmeldetag: 17.02.2020
(51) Int. Cl.: A61K 8/04, A61K 8/06, A61K 8/37, A61K 8/73, A61Q 19/00

(54) **MOUSSEARTIGE KOSMETISCHE ZUBEREITUNG**
MOUSSE-LIKE COSMETIC PREPARATION
PRÉPARATION COSMÉTIQUE SOUS FORME DE MOUSSE

(30) Priorität: 08.03.2019 DE 102019203184
(43) Veröffentlichungstag der Anmeldung: 10.11.2021
(73) Patentinhaber: Beiersdorf AG, 22529 Hamburg (DE)
(72) Erfinder: PESCHKE, Lisa, 22303 Hamburg (DE); RASCHKE, Thomas, 25421 Pinneberg (DE); CHANTY, Delphine, 37150 León (MX)
(74) Vertreter: Beiersdorf AG
(86) Internationale Anmeldenummer: PCT/EP2020/054035
(87) Internationale Veröffentlichungsnummer: WO 2020/182418

(56) Entgegenhaltungen:
- WO-A1-2017/048557
- CN-A- 107 955 749
- PL-B1- 229 611
- US-A1- 2016 368 186
- DATABASE GNPD [online] MINTEL; 17 June 2008 (2008-06-17), ANONYMOUS: "Dream Whip", XP055679660, retrieved from www.gnpd.com Database accession no. 932195

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Konventionelle kosmetische Produkte liegen oftmals als Feststoff, als Puder, als flüssiges Gel oder in Form einer Creme vor. Neben diesen bekannten Produktformen sind dem Fachmann ebenfalls aufgeschäumte kosmetische Zubereitungen bekannt.

Schäume bzw. Mousse sind Gebilde aus gas- oder luftgefüllten, kugel- oder polyederförmigen Zellen, welche durch flüssige, halbflüssige, hochviskose oder feste Zellstege begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum. Schäume sind thermodynamisch instabil, da durch Verkleinerung der Oberfläche Oberflächenenergie gewonnen werden kann. Die Stabilität und damit die Existenz eines Schaums ist somit davon abhängig, wieweit es gelingt, seine Selbstzerstörung zu verhindern.

Kosmetische Schäume sind in der Regel dispergierte Systeme aus Flüssigkeiten und Gasen, wobei die Flüssigkeit das Dispergiermittel und das Gas die dispergierte Substanz darstellen. Schäume aus niedrigviskosen Flüssigkeiten werden temporär durch oberflächenaktive Substanzen (Tenside, Schaumstabilisatoren) stabilisiert. Solche Tensidschäume haben aufgrund ihrer großen inneren Oberfläche ein starkes Adsorptionsvermögen, welches beispielsweise bei Reinigungs- und Waschvorgängen ausgenutzt wird. Dementsprechend finden kosmetische Schäume insbesondere in den Bereichen der Reinigung, beispielsweise als Rasierschaum, und der Haarpflege Verwendung.

Im Stand der Technik sind verschiedene Arten der Erzeugung von aufgeschäumten kosmetischen Zubereitungen bekannt. So wird üblicher Weise Gas in eine geeignete Flüssigkeiten eingeblasen, oder man erreicht die Schaumbildung durch heftiges Schlagen, Schütteln, Verspritzen oder Rühren der Flüssigkeit in der betreffenden Gasatmosphäre, vorausgesetzt, dass die Flüssigkeiten geeignete Tenside oder andere grenzflächenaktive Stoffe (sogenannte Schaumbildner) enthalten, die außer Grenzflächenaktivität auch ein gewisses Filmbildungsvermögen besitzen.

Aufgeschäumte kosmetische Zubereitungen haben gegenüber cremeförmigen oder gelartigen, flüssigen kosmetischen Zubereitungen den Vorteil, dass sie eine feine Verteilung von Wirkstoffen auf der Haut erlauben. Zudem schätzen die Verbraucher diese Form von kosmetischen Produkten, da durch die Gas- bzw. Lufteinschlüsse sich diese Produkte besonders leicht anfühlen und so effektiv verteilen lassen. Allerdings sind kosmetische Schäume in der Regel nur durch Verwendung besonderer Tenside zum Aufschäumen durch den Verbraucher oder durch Lagerung der kosmetischen Zubereitung unter Treibgas in einem Aerosolbehälter, welcher eine aufgeschäumte kosmetische Zubereitung ausgibt, zugänglich.

Aerosolprodukte zur Bereitstellung von mousseartigen / geschäumten Zubereitungen sind dem Fachmann unter anderem aus WO2013/080127 A2 bekannt. Jedoch weisen diese Aerosolprodukte eine Vielzahl von Nachteilen auf:
- So ist oftmals eine Lagerung unter Treibgas in einer Aerosoldose notwendig, welches einen Verkauf in einem gut handhabbaren Tiegel verhindert. Nur durch Ausgabe aus der Aerosoldose mit Treibgas kann ein Schaum oder Mousse erzeugt werden.
- Der Einsatz von Treibgasen in Aerosolbehältern ist in der Produktion teuer, da eine Aersolbefüllungsanlage für die Treibgase verwendet werden muss, statt einer einfachen Abfüllung in Tiegel.
- Mit Treibgasen aufgeschäumte kosmetische Zubereitungen weisen oftmals eine unzureichende Stabilität auf, so dass sich der Schaum oder das Mousse mit Lagerzeit verändert, bspw. zusammenfällt, so dass ein Aufschäumen in der Produktionsstätte und anschließender Verkauf als aufgeschäumte kosmetische Zubereitung nicht möglich ist.
- Weiterhin werden mousseartige Zubereitungen oftmals unter Schutzgas und nicht unter atmosphärischen Bedingungen hergestellt. Folglich entstehen auch hier erhöhte Produktionskosten.

PL 229611 B1 zeigt ein Verfahren, bei dem mousseartige Strukturen erzeugt werden sollen. Dazu wird Natriumhydrogencarbonat mit Zitronensäure in Gegenwart von Wasser reagiert und so CO₂ in die Formulierung eingeführt wird. Die Zubereitungen enthalten zusätzlich als Stabilisator Natriumcarboxymethylstärke.

CN10792249 A offenbart Emulsionen enthalten Natriumcarboxymethylstärke. Es ergeht kein Hinweis auf Schaumstrukturen.

WO2017/048557 A1 offenbart Formulierungen, die beim Austragen mit einer Pumpe geschäumt werden können.

US 2016/0368186 A1 offenbart geschäumte Packmittelt.

Unter der Mintel GNPD Eintragungsnummer 932195 ist das Produkt Redken Blonde Glam Dream Whip bekannt. Hier wird eine Zubereitung mittels eines Treibgases ausgetragen und aufgeschäumt.

Wünschenswert ist es kosmetische Zubereitungen bereitzustellen, welche derartige Nachteile nicht aufweisen. Insbesondere ist es wünschenswert kosmetische Zubereitungen bereitzustellen, welche mousseartige Strukturen, bzw. Schaumstrukturen ausbilden, ohne dass diese aus einem Aerosol mit Treibgas aufgeschäumt werden müssen. Weiterhin sollten die mousseartigen Zubereitungen besonders stabil sein, so dass diese auch über längere Zeiträume von 30 Tagen und mehr ihr Erscheinungsbild ohne Phasentrennung oder Zusammenfallen der Zubereitung erhalten. Weiterhin sollten die mousseartigen Zubereitungen ein mattes, nicht glänzendes Erscheinungsbild aufweisen und gleichzeitig wenig körnig wirken.

Überraschend wurde nun gefunden, dass die Zubereitungen gemäß der vorliegenden Erfindung mousseartige, bzw. Schaumstrukturen aufweisen, ohne dass diese mit Treibmitteln aus einem Aerosolbehälter oder durch Einrühren von Luft aufgeschäumt werden müssen.

Gegenstand der vorliegenden Erfindung ist eine kosmetische Emulsion enthaltend
a) Natriumcarboxymethylstärke und
b) Wasser;
dadurch gekennzeichnet, dass das Gewichtsverhältnis von Wasser zu Natriumcarboxymethylstärke weniger als 160:1 und mehr als 20:1 beträgt.

Auch Gegenstand der vorliegenden Erfindung ist die Verwendung von Natriumcarboxymethylstärke zur Ausbildung von Mousse- und/oder Schaumstrukturen in einer kosmetischen wasserhaltigen Emulsion, dadurch gekennzeichnet, dass das Gewichtsverhältnis von Wasser zu Natriumcarboxymethylstärke weniger als 160:1 und mehr als 20:1 beträgt.

Ohne an die Therorie gebunden zu sein, wird vermutet, dass die Gegenwart von Natriumcarboxymethylstärke in Emulsion dazu führt, dass nach Herstellung der Emulsion, Wasser von der Natriumcarboxymethylstärke gebunden wird. Dieses führt vermutlich zur Ausbildung von luftgefüllten Hohlräumen in der Zubereitung.

Überraschend war es weiterhin, dass im Gegensatz zu mit Treibgas aufgeschäumten Zubereitungen, die Zubereitungen bei Ausbildung der mit Luft gefüllten Hohlräume nicht ihr Volumen vergrößert. Somit konnte auch ein weiter Produktnachteil überraschend überwunden werden. Denn, wenn eine Zubereitung mit der Zeit ihr Volumen vergrößert, so muss diese in einem voluminöseren Packmittel verpackt werden. Stattdessen, kann für die Zubereitung der vorliegenden Erfindung ein Packmittel verwendet werden, welches dem Volumen der Zubereitung direkt nach Herstellung entspricht. Es muss kein zusätzlicher, unnötiger Platz für das Ausdehnen der Zubereitung berücksichtigt werden.

Ferner unterscheiden sich die erfindungsgemäßen Zubereitung und die aus diesen erhaltenen Mousse bzw. Schaumstrukturen von Schäumen die aus einer mit Treibgas aus einer Aerosoldose aufgeschäumten Zubereitung erhalten werden darin, dass eine gleichmäßigere, feinporigere Blasengröße erzielt wird.

Sollten Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen Emulsion.

Sollten Verhältnisse von Komponenten/Substanzen/Stoffgruppen offenbart werden, so beziehen sich diese Verhältnisse auf Gewichtsverhältnisse der genannten Komponenten/Substanzen/Stoffgruppen.

Werden nachfolgend Gewichtsprozentbereiche für die Bestandteile der kosmetischen Emulsion angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der vorliegenden Erfindung" etc. beziehen sich im Rahmen der vorliegenden Offenbarung immer auf die erfindungsgemäße Emulsion sowie auf die erfindungsgemäße Verwendung.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird der Begriff Haut verwendet, so bezieht dieser sich bevorzugt auf die menschliche Haut.

Es ist erfindungsgemäß von Vorteil, wenn das Gewichtsverhältnis von Wasser zu Natriumcarboxymethylstärke weniger als 100:1, bevorzugt weniger als 80:1 beträgt und insbesondere bevorzugt weniger als 60:1.

Weiterhin ist es erfindungsgemäß von Vorteil, wenn das Gewichtsverhältnis von Wasser zu Natriumcarboxymethylstärke bevorzugt mehr als 30:1 beträgt.

Folglich sind erfindungsgemäß vorteilhafte Gewichtsverhältnisse von Wasser zu Natriumcarboxymethylstärke im Bereich von 80:1 bis 20:1 und insbesondere bevorzugt von 60:1 bis 30:1.

Überraschend wurde gefunden, dass zusätzlich die Schaum bzw. Mousse-Eigenschaften durch Auswahl bestimmten Gewichtsverhältnisse von Wasser zu Natriumcarboxymethylstärke beeinflusst werden können. So wurden die folgend Aspekte überraschend festgestellt:
Beträgt das Gewichtsverhältnis von Wasser zu Natriumcarboxymethylstärke von 80:1 bis 10:1, so wurden grobblasige Mousse-/Schaumstrukturen gefunden. Die gleichmäßigste Verteilung der Luftblasen wird bei einem Verhältnis von 60:1 bis 30:1.

Wird ein Gewichtsverhältnis von Wasser zu Natriumcarboxymethylstärke von weniger als 10:1 gewählt, das heißt mit zunehmen Anteil Natriumcarboxymethylstärke im Vergleich zu Wasser, so werden überraschend weniger sichtbare Luftblasen in der Zubereitung erhalten. Die Zubereitungen enthaltend einen höheren Anteil von Wasser im Vergleich zum Anteil von Natriumcarboxymethylstärke, bzw. ein Gewichtsverhältnis von mehr als 10:1 von Wasser zu Carboxymethylstärke, zeigen größere deutlich sichtbarere Luftblasen. Entsprechend wird bei diesen Zubereitungen eine deutlich wahrnehmbarere Schaumstruktur erhalten. Die Zubereitung entspricht eher einer Mousse, wie sie beispielsweise aus Lebensmittelprodukten bekannt ist.

Andere Stärken, wie beispielsweise Tapiokastärke, zeigen bei alleinigem Einsatz keine Ausbildung einer Mousse oder Schaumstrukturen.

Somit war es überraschend möglich Schaum oder Moussestrukturen in einer kosmetischen Emulsion auszubilden, ohne dass aktiv Treibgas in die Emulsion eingeblasen werden muss, oder dass die Emulsion in einem Aerosolbehälter mit Treibgas verpackt wird und aus diesem ausgegeben wird.

Überraschend fiel die Schaum und/oder Moussestruktur der erfindungsgemäßen Emulsion auch bei Lagerung unter Normalbedingungen über 30 Tage nicht zusammen.

Weiterhin wurde überraschend gefunden, dass erfindungsgemäße Emulsionen mit einem Gewichtsverhältnis von Wasser zu Natriumcarboxymethylstärke mit weniger als 50:1, das heißt mit abnehmenden Anteil von Wasser im Verhältnis zu Natriumcarboxymethylstärke, eine besonders matte Oberfläche aufweisen. Dieses ist analytisch mit einem UV/VIS Spektrometer mit Ulbricht-Kugel nachweisbar. So wurde gefunden, dass nach Einstrahlung überraschend weniger gerichtete Streuung messbar ist, als bei Emulsionen mit einem höheren Wasseranteil in Referenz zu Natriumcarboxymethylstärke.

Ferner sind die Emulsionen mit einem Gewichtsanteil von Wasser zu Natriumcarboxymethylstärke von 70:1 bis 20:1, bevorzugt von 60:1 bis 30:1, überraschend besonders leicht streichfähig und lassen sich leicht auf der Haut verteilen. Diese Emulsionen fühlen sich überraschend besonders leicht und cremig an. Wird ein höherer Wassergehalt in Referenz zu Natriumcarboxymethylstärke gewählt, so fühlt sich die Emulsion klumpig bis griesig auf der Haut an. Wird ein niedrigerer Gewichtsanteil von Wasser in Referenz zu Natriumcarboxymethylstärke gewählt, so waren die Emulsionen zähflüssiger und nicht leicht auf der Haut verteilbar. Das leichte Gefühl der mousseartigen Emulsion ging verloren.

Der Anteil von Wasser in der erfindungsgemäßen Emulsion beträgt 55 bis 90 Gew.-%, bevorzugt von 60 bis 80 Gew.-% und insbesondere bevorzugt von 65 bis 75 Gew.-%, bezogen auf das Gesamtgewicht der Emulsion.

Der Anteil von Natriumcarboxymethylstärke in der erfindungsgemäßen Emulsion beträgt 0,5 bis 6 Gew.-%, bevorzugt von 1 Gew.-% bis 4 Gew.-% und insbesondere bevorzugt von 1,5 bis 3,5 Gew.-%, bezogen auf das Gesamtgewicht der Emulsion.

Die nachfolgend beschriebenen Merkmale der erfindungsgemäßen Emulsion beziehen sich auf die erfindungsgemäße Emulsion an sich, sowie jeweils auf die erste und zweite vorteilhafte Ausführungsform der Erfindung.

Natriumcarboxymethylstärke mit der CAS-Nr. 9063-38-1 trägt die INCI-Bezeichnung Sodium carboxy methyl starch. Es ist erfindungsgemäß bevorzugt, wenn die Natriumcarboxymethylstärke auf Kartoffelstärke basiert. Es ist insbesondere bevorzugt, neben Kartoffelstärke keine weiten Stärken zur Herstellung der Natriumcarboxymethylstärke eingesetzt werden.

Ein Beispiel für eine erfindungsgemäße Natriumcarboxymethylstärke auf Basis von Kartoffelstärke ist unter der Handelsbezeichnung COVAGEL von der Fa. LCW bekannt. Weitere erfindungsgemäße Natriumcarboxymethylstärken können unter den Handelsnamen GLYCOLYS von der Firma Roquette sowie VIVASTAR P3500 und VIVASTAR CS INSTANT Powder von JRS Pharma bezogen werden.

Es ist erfindungsgemäß generell von Vorteil, wenn die Emulsion keine Acrylat-basierten Polymere enthält. Acrylat-basierte Polymere sind Polymere, welche aus Homo- oder Copolymerisation mit Acryl- und/oder Methacrylsäure erhalten werden. Beispiele hierfür sind u.a. Carbomer oder Acrylate Copolymer. Acrylat-basierte Polymere werden oftmals kritisch in der Öffentlichkeit hinsichtlich ihrer Umweltverträglichkeit diskutiert. Entsprechend sprechen kosmetische Produkte enthaltend Acrylat-basierte Polymere nicht mehr alle Konsumentengruppen an. Darüber war es überraschend für den Fachmann, dass ich durch die erfindungsgemäße Emulsion mousseartige Strukturen ausbilden lassen, wenn die Emulsion keine Acrylat-basierten Polymere enthält.

Ferner ist es vorteilhaft, wenn neben Natriumcarboxymethylstärke mindestens ein weiteres Polymer auf Basis von Polysacchariden mit Ausnahme von Polysacchariden, welche mit Acrylsäure oder Methacrylsäure modifiziert wurden, enthalten ist. Derartige bevorzugte Polymere sind unter anderem Polymere auf Basis von Cellulosen, insbesondere bevorzugt gewählt aus der Gruppe Carboxymethylcellulose, Hydroxyethylcellulose, Hydroxypropylcellulose, Ethylcellulose, Methylcellulose, Ethyl Hydroxyethyl Cellulose und Methyl Ethyl Cellulose.

Weitere bevorzugte Polymere sind gewählt aus der Gruppe der Polymere auf Basis von Stärken, insbesondere bevorzugt gewählt aus der Gruppe Hydroxypropyl Starch Phosphate, Sodium Hydroxypropyl Starch Phosphate, Hydroxypropyl Starch Phosphate und Distarch Phosphate.

Ein weiteres bevorzugtes Polymer ist Xantham Gum.

Weitere bevorzugte Polymere sind Sclerotium Gum, Locust Bean Gum und / oder Gellan Gum.

Es ist ferner bevorzugt, wenn der Gesamtanteil der weiteren Polymer auf Basis von Polysacchariden mit Ausnahme von Polysacchariden, welche mit Acrylsäure oder Methacrylsäure modifiziert wurden, von 0,1 bis 8 Gew.-%, bevorzugt 0,2 bis 5 Gew.-% bezogen auf das Gesamtgewicht der Emulsion beträgt.

Es ist insbesondere vorteilhaft, wenn Xanthan Gum, Sclerotium Gum, Locust Bean Gum und / oder Gellan Gum in einem Anteil von 0,05 bis 0,5 Gew.-% in der Emulsion enthalten ist, wobei sich die Angabe auf das Gesamtgewicht der Emulsion bezieht.

Vorteilhaft ist die Emulsion der vorliegenden Erfindung dadurch gekennzeichnet, dass diese eine Öl-in-Wasser Emulsion ist.

Weiterhin ist es vorteilhaft, wenn der Anteil der Ölphase von 3 bis 20 Gew.-%, bevorzugt von 5 bis 18 Gew.-%, weiterhin bevorzugt von 6 bis 15 Gew.-% und insbesondere bevorzugt von 7 bis 12 Gew.-% beträgt, wobei Emulgatoren und Tenside definitionsgemäß nicht zur Ölphase zählen und sich die Angaben auf das Gesamtgewicht der Emulsion beziehen.

Unter Emulgatoren werden alle Substanzen verstanden, welche im International Cosmetic Ingredient Dictionary and Handbook, Thirteenth Edition 2010, (ISBN 1-882621-47-6) unter der Bezeichnung "emulsifying agent" geführt werden. Unter Tensiden werden alle Substanzen verstanden, welche im International Cosmetic Ingredient Dictionary and Handbook, Thirteenth Edition 2010, (ISBN 1-882621-47-6) unter der Bezeichnung " surfactant " geführt werden.

Ferner ist es vorteilhaft, wenn die Ölphase, respektive die Emulsion, Caprylic/Capric Triglyceride enthält. Ist Caprylic/Capric Triglyceride in der Emulsion enthalten, so beträgt der Anteil von Caprylic/Capric Triglyceride vorteilhaft von 6 bis 13 Gew.-% und bevorzugt von 7,5 bis 11 Gew.-%, bezogen auf das Gesamtgewicht der Emulsion.

Weiterhin ist es vorteilhaft, wenn die Ölphase, respektive die Emulsion, gehärtetes Kokosöl (INCI: hydrogenated coco glycerides) enthält. Ist gehärtetes Kokosöl in der Emulsion enthalten, so beträgt der Anteil von gehärtetes Kokosöl vorteilhaft von 0,4 bis 3 Gew.-% bezogen auf das Gesamtgewicht der Emulsion.

Weiterhin ist es vorteilhaft im Sinne der vorliegenden Erfindung, wenn die Emulsion der vorliegenden Erfindung ein oder mehrere Ester aus einwertigen Alkoholen und Monocarbonsäuren enthält, wobei die Ester mindestens 10, bevorzugt mindestens 15 Kohlenstoffatome aufweisen. Vorteilhaft zu wählende Ester aus einwertigen Alkoholen und Monocarbonsäuren sind 2-Ethylhexylisostearat, Isotridecylisononanoat, 2-Ethylhexylcocoat, C₁₂₋₁₅-Alkylbenzoat, Isopropylmyristat und Isopropylpalmitat.

Ferner ist es vorteilhaft im Sinne der vorliegenden Erfindung, wenn die Emulsion der vorliegenden Erfindung ein oder mehrere Dialkylether mit 12 bis 24 Kohlenstoffatomen enthält, wobei bevorzugt Dicaprylylether enthalten ist. Enthält die Emulsion Dicaprylylether, so beträgt der Anteil von Dicaprylylether bevorzugt von 0,5 bis 3 Gew.-%, bezogen auf das Gesamtgewicht der Emulsion.

Weiterhin können die erfindungsgemäßen Emulsionen vorteilhaft pflanzliche Öle, wie bevorzugt Sonnenblumenöl (Helianthus Annuus Seed Oil), Rapsöl (Canola Oil), Sojaöl (Glycine Soja Oil), Olivenöl (Olea Europaea Fruit Oil), Mandelöl (Prunus amygdalus dulcis Oil), Avocadoöl (Persea Gratissima Oil), Walnussöl (Junglans Regia Seed Oil), Pfirsichkernöl (Prunus Persica Kernel Oil), Aprikosenkernöl (Prunus Armeniaca Kernel Oil), Sesamöl (Sesamum Indicum Seed Oil), Camelienöl (Camelia Oleifera/ Camelia Sasanqua), Nachtkerzenöl (Oenothera biennis), Macadamianussöl (Macadamia Intergrifolia Seed Oil), Diestelöl (Silybum Marianum Seed Oil), Weizenkeimöl (Triticum Vulgare Germ Oil), Palmkernöl (Elaeis guineensis Kernel Oil), Palmöl (Elaeis guineensis Oil), Traubenkernöl (Vitis Vinifera Seed Oil), Arganöl (Argania spinosa Seed Oil), Erdnussöl (Arachis hypogaea Oil), Kürbiskernöl (Cucurbita Pepo Seed Oil), Ricinusöl (Ricinus Communis Seed Oil), Reiskeimöl (Oryza Sativa Bran Oil), Vegetable Oil (Olus Oil) und/oder Kokosöl, enthalten.

Es ist auch vorteilhaft, wenn die Emulsion der vorliegenden Erfindung ein oder mehrere verzweigte und/oder unverzweigte Kohlenwasserstoffe enthält, sofern diese nicht gasförmig unter Normalbedingungen sind. Vorteilhaft zu verwendende Kohlenwasserstoffe sind Paraffinum Liquidum, Isododecan, Isohexadecan, Isoeicosane, Squalan und Cera Microcristallina.

Vorteilhaft enthält die Emulsion der vorliegenden Erfindung mindestens einen Emulgator.

Unter den bekannten Emulgatoren sind bevorzugt Glyceryl Stearate, Glyceryl Stearate Citrate, Polyglyceryl-10 Stearate und/oder Fettsäuren aufweisend 14 bis 22 Kohlenstoffe und/oder deren Salze zu wählen. Insbesondere der Einsatz von Polyglyceryl-10 Stearate und/oder Fettsäuren aufweisend 14 bis 22 Kohlenstoffe und/oder deren Salze führte zu Emulsionen, welche bei Lagerung unter 40°C keine Phasenseparation zeigten.

Weiterhin ist es vorteilhaft im Sinne der Erfindung, wenn der Gesamtanteil von Polyglyceryl-10 Stearate und Fettsäuren aufweisend 14 bis 22 Kohlenstoffe und/oder deren Salze von 0,1 bis 5 Gew.-%, bevorzugt von 0,5 bis 4 Gew.-% und insbesondere bevorzugt von 1 bis 3 Gew.-% bezogen auf das Gesamtgewicht der Emulsion beträgt.

Somit beträgt der Gesamtanteil der Emulgatoren vorteilhaft von 0,1 bis 10 Gew.-%, bevorzugt von 0,5 bis 8 Gew.-% und insbesondere bevorzugt von 1 bis 6 Gew.-% bezogen auf das Gesamtgewicht der Emulsion.

Weitere vorteilhafte Emulsionen enthalten die Emulgatoren vorteilhaft in einem Gesamtanteil von 0,1 bis 5 Gew.-%, bevorzugt von 0,5 bis 4 Gew.-% und insbesondere bevorzugt von 1 bis 3 Gew.-% bezogen auf das Gesamtgewicht der Emulsion.

Es ist in einer Ausführungsform insbesondere vorteilhaft, wenn die Emulsion ein oder mehre Fettsäuren aufweisend 14 bis 22 Kohlenstoffatome und/oder deren Salze in einem Anteil von 0,1 bis 5 Gew.-%, bevorzugt von 0,5 bis 4 Gew.-% und insbesondere bevorzugt von 1 bis 3 Gew.-% bezogen auf das Gesamtgewicht der Emulsion, enthält. Innerhalb dieser Ausführungsform ist es bevorzugt, wenn mindestens Stearinsäure und/oder Palmitinsäure oder deren Salze enthalten sind.

Es ist in einer weiteren Ausführungsform insbesondere vorteilhaft, wenn die Emulsion Polyglyceryl-10 Stearate in einem Anteil von 0,1 bis 5 Gew.-%, bevorzugt von 0,5 bis 4 Gew.-% und insbesondere bevorzugt von 1 bis 3 Gew.-% bezogen auf das Gesamtgewicht der Emulsion, enthält.

Es ist ebenfalls vorteilhaft im Sinne der vorliegenden Erfindung, wenn die kosmetische Emulsion Glyceryl Stearate enthält. Enthält die Emulsion Glyceryl Stearate, so ist es bevorzugt, wenn der Anteil von Glyceryl Stearate von 0,1 bis 3 Gew.-%, weiterhin bevorzugt von 0,3 bis 2,5 Gew.-% und insbesondere bevorzugt 0,5 bis 1,5 Gew.-% bezogen auf das Gesamtgewicht der Emulsion beträgt.

Weiterhin ist es vorteilhaft, wenn die erfindungsgemäße Emulsion Glyceryl Stearate Citrate in einem Anteil von 0,5 bis 4 Gew.-%, bevorzugt 1 bis 3 Gew.-% und insbesondere bevorzugt 1,5 bis 2,5 Gew.-%, bezogen auf das Gesamtgewicht der Emulsion, enthält.

Weiterhin ist es vorteilhaft, wenn die kosmetische Emulsion der Erfindung mindestens einen Fettalkohol, insbesondere Stearylalkohol, Cetylalkohol und/oder Behenylalkohol, enthält. Erfindungsgemäße Mischungen aus Stearylalkohol und Cetylalkohol sind unter der INCI Bezeichnung Ceteraylalkohol bekannt. Sofern die kosmetische Emulsion mindestens einen Fettalkohol, insbesondere Stearylalkohol, Cetylalkohol und/oder Behenylalkohol, enthält, so ist es bevorzugt wenn der Anteil der Fettalkohole von 0,5 bis 4 Gew.-% bezogen auf das Gesamtgewicht der Emulsion beträgt. Es ist insbesondere vorteilhaft, wenn Stearylalkohol, Cetylalkohol und/oder Behenylalkohol in einem Anteil von 1 bis 4 Gew.-% bezogen auf das Gesamtgewicht der Emulsion enthalten sind, wobei es weiterhin bevorzugt ist, wenn keine weiten Fettalkohole enthalten sind. Der Zusatz der oben genannten Fettalkohole führt überraschend zu einer weiteren Reduktion der Blasengröße und somit zu einer feineren bzw. feinblasigeren Mousse-/ Schaumstruktur. Insbesondere vorteilhafte und überraschende Ergebnisse wurden erzielt, wenn Behenylalkohol enthalten ist und der Anteil von Behenylalkohol von 1,5 Gew.-% bis 4 Gew.-%, bevorzugt 2 Gew.-% bis 3,5 Gew.-%, bezogen auf das Gesamtgewicht der Emulsion, beträgt.

Es ist ebenfalls vorteilhaft, wenn die kosmetische Emulsion Ethanol enthält, wobei der Anteil von Ethanol bevorzugt von 0,5 bis 5 Gew.-%, insbesondere bevorzugt von 1 bis 3 Gew.-% bezogen auf das Gesamtgewicht der Emulsion beträgt.

Weiterhin sind vorteilhafte Emulsionen dadurch gekennzeichnet, dass diese Butylene Glycol enthalten, wobei der Anteil von Butylene Glycol bevorzugt von 1 bis 8 Gew.-% und weiterhin bevorzugt von 2 bis 5 Gew.-% und insbesondere bevorzugt von 2,5 bis 4 Gew.-% bezogen auf das Gesamtgewicht der Emulsion beträgt.

Es ist darüber hinaus erfindungsgemäß von Vorteil, wenn die Emulsion Propylenglycol, 2-Methylpropan-1,3-diol, 1,2-Pentandiol, 1,2-Hexandiol, 1,2-Octandiol Glycerylcaprylate und/oder 1,2-Decandiol enthält.

Weiterhin ist es ebenfalls vorteilhaft im Sinne der vorliegenden Erfindung, wenn die Emulsion Phenoxyethanol, Methylparaben, Ethylparaben, Sorbinsäure und ihre Alkalisalze, Dehydracetsäure, Benzethoniumchlorid und/oder Ethylhexylglycerin enthält.

Enthält die Emulsion Phenoxyethanol, so ist es bevorzugt, wenn der Anteil an Phenoxyethanol von 0,2 bis 1,0 Gew.-% beträgt, bezogen auf das Gesamtgewicht der Emulsion.

Enthält die Emulsion Methylparaben und/oder Ethylparaben, so ist es bevorzugt, wenn der Gesamtanteil an Methylparaben und/oder Ethylparaben von 0,01 bis 0,5 Gew.-% beträgt, bezogen auf das Gesamtgewicht der Emulsion.

Nicht zuletzt sind erfindungsgemäß vorteilhafte Ausführungsformen dadurch gekennzeichnet, dass die Emulsion einen oder mehrere Wirkstoffe gewählt aus der Gruppe der Verbindungen Glycyrrhetinsäure, Harnstoff, Arctiin, Folsäure, Coenzym Q10 (Ubiquinon), alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat Vitamin C, Vitamin C phosphate, Vitamin C palmitate, Niacinamide, Vitamin A palmitate, Retinol, Panthenol, Licochalcon A, Rucinol, N-[(2,4-Dihydroxyphenyl)thiazol-2-yl]isobutyramide, Honociol und Magnolol (auch als Bestandteil von Magnolia-Extrakten), Hyaluronsäure und/oder Silymarin (Mariendistelextrakt) enthält.

In einer Ausführungsform der vorliegenden Erfindung ist es vorteilhaft, wenn die Emulsion keine Silikonöle oder Silikonhaltige Substanzen enthält.

Eine weitere Ausführungsform der Erfindung ist vorteilhaft dadurch gekennzeichnet, dass die Emulsion mindestens ein Silikonöl enthält.

Es ist ferner vorteilhaft, wenn die erfindungsgemäße Emulsion Glycerin in einem Anteil von 0,5 bis 12 Gew.-%, bevorzugt von 2 bis 10 Gew.-%, bezogen auf das Gesamtgewicht der Emulsion enthält.

Die erfindungsgemäßen kosmetische Emulsion kann ferner Farbstoffe und / oder Farbpigmente enthalten, insbesondere wenn sie die Emulsion zur dekorativer Kosmetik genutzt wird. Die Farbstoffe und Farbpigmente können aus der entsprechenden Positivliste der Kosmetikrichtlinie oder der EG-Liste kosmetischer Farbstoffe ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe 2 O 3, Fe 3 O 4, FeO (OH)) und / oder Zinnoxid. Vorteilhafte Farbstoffe sind beispielsweise Carmin, Berliner Blau, Chromoxidgrün, Ultramarinblau und / oder Manganviolett. Es ist besonders vorteilhaft, die Farbstoffe und / oder Farbpigmente aus der folgenden Liste auszuwählen. Die Farbindexnummern (CIN) stammen aus dem Rowe Colour Index, 3. Auflage, Society of Dyers and Colourists, Bradford, England, 1971.

| **Inhaltstoff** | **CIN** | **Farbe** |
|---|---|---|
| Piqment Green | 10006 | qrün |
| Acid Green 1 | 10020 | qrün |
| 2,4-Dinitrohydroxynaphthalene-7-sulfonic acid | 10316 | gelb |
| Piqment Yellow 1 | 11680 | gelb |
| Piqment Yellow 3 | 11710 | gelb |
| Piqment Orange 1 | 11725 | orange |
| 2,4-Dihydroxyazobenzene | 11920 | orange |
| Solvent Red 3 | 12010 | rot |
| 1-(2'-Chloro-4'-nitro-1'-phenylazo)-2-hydroxynaphthalene | 12085 | rot |
| Piqment Red 3 | 12120 | rot |
| Ceres red; Sudan red; Fat Red G | 12150 | rot |
| Piqment Red 112 | 12370 | rot |
| Piqment Red 7 | 12420 | rot |
| Piqment Brown 1 | 12480 | braun |
| 4-(2'-Methoxy-5'-sulfodiethylamido-1'-phenylazo)-3-hydroxy-5"-chloro-2",4"-dimethoxy-2-naphthanilide | 12490 | rot |
| Disperse Yellow 16 | 12700 | gelb |
| 1-(4-Sulfo-1-phenylazo)-4-aminobenzene-5-sulfonic acid | 13015 | gelb |
| 2,4-Dihydroxyazobenzene-4'-sulfonic acid | 14270 | orange |
| 2-(2,4-Dimethylphenylazo-5-sulfo)-1-hydroxynaphthalene-4-sulfonic acid | 14700 | rot |
| 2-(4-Sulfo-1-naphthylazo)-1-naphthol-4-sulfonic acid | 14720 | rot |
| 2-(6-Sulfo-2,4-xylylazo)-1-naphthol-5-sulfonic acid | 14815 | rot |
| 1-(4'-Sulfophenylazo)-2-hydroxynaphthalene | 15510 | orange |
| 1-(2-Sulfo-4-chloro-5-carboxy-1-phenylazo)-2-hydroxynaphthalene | 15525 | rot |
| 1-(3-Methylphenylazo-4-sulfo)-2-hydroxynaphthalene | 15580 | rot |
| 1-(4',(8')-Sulfonaphthylazo)-2-hydroxynaphthalene | 15620 | rot |
| 2-Hydroxy-1,2'-azonaphthalene-1'-sulfonic acid | 15630 | rot |
| 3-Hydroxy-4-phenylazo-2-naphthylcarboxylic acid | 15800 | rot |
| 1-(2-Sulfo-4-methyl-1-phenylazo)-2-naphthylcarboxylic acid | 15850 | rot |
| 1-(2-Sulfo-4-methyl-5-chloro-1-phenylazo)-2-hydroxynaphthalene- 3-carboxylic acid | 15865 | rot |
| 1-(2-Sulfo-1-naphthylazo)-2-hydroxynaphthalene-3-carboxylic acid | 15880 | rot |
| 1-(3-Sulfo-1-phenylazo)-2-naphthol-6-sulfonic acid | 15980 | orange |
| 1-(4-Sulfo-1-phenylazo)-2-naphthol-6-sulfonic acid | 15985 | gelb |
| Allura Red | 16035 | rot |
| 1-(4-Sulfo-1-naphthylazo)-2-naphthol-3,6-disulfonic acid | 16185 | rot |
| Acid Orange 10 | 16230 | orange |
| 1-(4-Sulfo-1-naphthylazo)-2-naphthol-6,8-disulfonic acid | 16255 | rot |
| 1-(4-Sulfo-1-naphthylazo)-2-naphthol-3,6,8-trisulfonic acid | 16290 | rot |
| 8-Amino-2-phenylazo-1-naphthol-3,6-disulfonic acid | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolone-3-carboxylic acid | 19140 | gelb |
| Piqment Yellow 16 | 20040 | gelb |
| 2,6-(4'-Sulfo-2", 4"-dimethyl)bisphenylazo)-1,3-dihydroxybenzene | 20170 | orange |
| Acid Black 1 | 20470 | schwarz |
| Piqment Yellow 13 | 21100 | gelb |
| Piqment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1 "-phenylazo)-7'-sulfo-1'-naphthylazo]-1-hydroxy-7-aminonaphthalene-3,6-disulfonic acid | 27755 | schwarz |
| 4'-[(4"-Sulfo-1"-phenylazo)-7'-sulfo-1'-naphthylazo]-1-hydroxy-8-acetylaminonaphthalene-3,5-disulfonic acid | 28440 | schwarz |
| Direct Orange 34, 39, 44, 46, 60 | 40215 | orange |
| Food Yellow | 40800 | orange |
| trans-β-Apo-8'-carotenaldehyde (C₃₀) | 40820 | orange |
| trans-Apo-8'-carotenic acid (C₃₀)-ethyl ester | 40825 | orange |
| Canthaxanthin | 40850 | orange |
| Acid Blue 1 | 42045 | blau |
| 2,4-Disulfo-5-hydroxy-4'-4"-bis(diethylamino)triphenylcarbinol | 42051 | blau |
| 4-[(4-N-Ethyl-p-sulfobenzylamino)phenyl(4-hydroxy-2-sulfophenyl)(methylene)-1-(N-ethyl-N-p-sulfobenzyl)-2,5-cyclohexadienimine] | 42053 | grün |
| Acid Blue 7 | 42080 | blau |
| (N-Ethyl-p-sulfobenzylamino)phenyl(2-sulfophenyl)methylene-(N-ethyl-N-p-sulfobenzyl)Δ^{2,5}-cyclohexadienimine | 42090 | blau |
| Acid Green 9 | 42100 | qrün |
| Diethyldisulfobenzyl-di-4-amino-2-chloro-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)amino-4"-(N-diethyl)amino-2-methyl-N-ethyl-N-m-sulfobenzylfuchsonimmonium | 42735 | blau |
| 4'-(N-Dimethyl)amino-4"-(N-phenyl)aminonaphtho-N-dimethyl-fuchsonimmonium | 44045 | blau |
| 2-Hydroxy-3,6-disulfo-4,4'-bisdimethylaminonaphtho-fuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)xanthenium salt | 45190 | violett |
| Acid Red 50 | 45220 | rot |
| Phenyl-2-oxyfluorone-2-carboxylic acid | 45350 | gelb |
| 4,5-Dibromofluorescein | 45370 | orange |
| 2,4,5,7-Tetrabromofluorescein | 45380 | rot |
| Solvent Dye | 45396 | orange |
| Acid Red 98 | 45405 | rot |
| 3',4',5',6'-Tetrachloro-2,4,5,7-tetrabromofluorescein | 45410 | rot |
| 4,5-Diiodofluorescein | 45425 | rot |
| 2,4,5,7-Tetraiodofluorescein | 45430 | rot |
| Quinophthalone | 47000 | gelb |
| Quinophthalonedisulfonic acid | 47005 | gelb |
| Acid Violet 50 | 50325 | violett |
| Acid Black 2 | 50420 | schwarz |
| Piqment Violet 23 | 51319 | violett |
| 1,2-Dioxyanthraquinone, calcium-aluminum complex | 58000 | rot |
| 3-Oxypyrene-5,8,10-sulfonic acid | 59040 | grün |
| 1-Hydroxy-4-N-phenylaminoanthraquinone | 60724 | violett |
| 1-Hydroxy-4-(4'-methylphenylamino)anthraquinone | 60725 | violett |
| Acid Violet 23 | 60730 | violett |
| 1,4-Di(4'-methylphenylamino)anthraquinone | 61565 | grün |
| 1,4-Bis(o-sulfo-p-toluidino)anthraquinone | 61570 | qrün |
| Acid Blue 80 | 61585 | blau |
| Acid Blue 62 | 62045 | blau |
| N,N'-Dihydro-1,2,1',2'-anthraquinone azine | 69800 | blau |
| Vat Blue 6; Piqment Blue 64 | 69825 | blau |
| Vat Orange 7 | 71105 | orange |
| Indiqo | 73000 | blau |
| Indiqo-disulfonic acid | 73015 | blau |
| 4,4'-Dimethyl-6,6'-dichlorothioindigo | 73360 | rot |
| 5,5'-Dichloro-7,7'-dimethylthioindigo | 73385 | violett |
| Quinacridone Violet 19 | 73900 | violett |
| Piqment Red 122 | 73915 | rot |
| Piqment Blue 16 | 74100 | blau |
| Phthalocyanine | 74160 | blau |
| Direct Blue 86 | 74180 | blau |
| Chlorinated phthalocyanine | 74260 | grün |
| Natural Yellow 6,19; Natural Red 1 | 75100 | gelb |
| Bixin, Norbixin | 75120 | orange |
| Lycopene | 75125 | gelb |
| trans-alpha-, beta- and gamma-carotene | 75130 | orange |
| Keto- and/or hydroxyl derivates of carotene | 75135 | gelb |
| Guanine or pearlescent aqent | 75170 | weiß |
| 1,7-Bis(4-hydroxy-3-methoxyphenyl)-1,6-heptadiene-3,5-dione | 75300 | gelb |
| Complex salt (Na, Al, Ca) of carminic acid | 75470 | rot |
| Chlorophyll a and b; copper compounds of chlorophylls and chlorophyllins | 75810 | grün |
| Aluminum | 77000 | weiß |
| Hydrated alumina | 77002 | weiß |
| Hydrous aluminum silicates | 77004 | weiß |
| Ultramarine | 77007 | blau |
| Piqment Red 101 and 102 | 77015 | rot |
| Barium sulfate | 77120 | weiß |
| Bismuth oxychloride and its mixtures with mica | 77163 | weiß |
| Calcium carbonate | 77220 | weiß |
| Calcium sulfate | 77231 | weiß |
| Carbon | 77266 | schwarz |
| Piqment black 9 | 77267 | schwarz |
| Carbo medicinalis veqetabilis | 77268:1 | schwarz |
| Chromium oxide | 77288 | qrün |
| Chromium oxide, hydrous | 77289 | qrün |
| Piqment Blue 28, Piqment Green 14 | 77346 | qrün |
| Piqment Metal 2 | 77400 | braun |
| Gold | 77480 | braun |
| Iron oxides and hydroxides | 77489 | orange |
| Iron oxide | 77491 | rot |
| Hydrated iron oxide | 77492 | gelb |
| Iron oxide | 77499 | schwarz |
| Mixtures of iron (II) and iron(III)hexacyanoferrate | 77510 | blau |
| Piqment White 18 | 77713 | weiß |
| Manqanese animonium diphosphate | 77742 | violett |
| Manqanese phosphate; Mn₃(PO₄)₂ · 7 H20 | 77745 | rot |
| Silver | 77820 | weiß |
| Titanium dioxide and its mixtures with mica | 77891 | weiß |
| Zinc oxide | 77947 | weiß |
| 6,7-Dimethyl-9-(1'-D-ribityl)isoalloxazine, lactoflavine | | gelb |
| Suqar colorinq | | braun |
| Capsanthin, capsorubin | | orange |
| Betanin | | rot |
| Benzopyrylium salts, anthocyans | | rot |
| Aluminum, zinc, maqnesium and calcium stearate | | weiß |
| Bromothymol blue | | blau |
| Bromocresol qreen | | qrün |
| Acid Red 195 | | rot |
| Silica-treated Red Iron Oxide | 77491 | rot |
| Silica-treated Black Iron Oxide | 77499 | schwarz |
| Silica-treated Yellow Iron Oxide | C338073 | gelb |
| Silica-treated Pigmentary Titanium Dioxide | 77891 | weiß |
| Alqinate-treated Red Iron Oxide | | rot |
| Alqinate-treated Black Iron Oxide | | schwarz |
| Alqinate-treated Yellow Iron Oxide | | gelb |
| Alqinate-treated Pigmentary Titanium Dioxide | | weiß |
| Sodium glycerophosphate-treated Iron Oxide | 77491 | rot |
| Sodium glycerophosphate-treated Iron Oxide | 77499 | schwarz |
| Sodium glycerophosphate-treated Iron Oxide | 77492 | gelb |
| Sodium glycerophosphate- treated Pigmentary Titanium Dioxide | 77891 | weiß |
| Hydrogenated-Lecithin-treated Iron Oxide | 77491 | rot |
| Hydrogenated-Lecithin-treated Iron Oxide | 77499 | schwarz |
| Hydrogenated-Lecithin-treated Iron Oxide | 77492 | gelb |
| Hydrogenated-Lecithin-treated Pigmentary Titanium Dioxide | 77891 | weiß |

Der Ausdruck "treated" in der vorstehenden Tabelle bedeutet im Sinne der vorliegenden Offenbarung, dass ein Pigment mit der jeweiligen Substanz behandelt wurde, so dass an der Oberfläche des Pigments sich die jeweils eingesetzte Substanz ablagert.

Vorteilhafte Emulsionen sind dadurch gekennzeichnet, dass diese 0,1 bis 12 Gew.-%, bevorzugt 3 bis 10 Gew.-% und insbesondere bevorzugt 5 bis 9 Gew.-% anorganische Pigmente enthalten, wobei sich die Angabe auf das Gesamtgewicht der Emulsion bezieht. Es ist insbesondere vorteilhaft, wenn mindestens ein anorganisches Pigment aufweisend ein Coating enthalten ist.

Eine vorteilhafte Ausführungsform der Erfindung ist dadurch gekennzeichnet, dass der Anteil der anorganischen Pigmente von 5 bis 10 Gew.-% bezogen auf das Gesamtgewicht der Emulsion beträgt, wobei der Anteil der gecoateten anorganischen Pigmente mindestens 60 Gew.-%, bevorzugt mindestens 90 Gew.-% bezogen auf das Gesamtgewicht aller enthaltenen anorganischen Pigment beträgt. Bevorzugt sind insbesondere die gecoateten Pigmente, die mit Silicat, Alginate, Sodium Glycerophosphate oder mit hydrogeniertem Lecithin beschichtet sind.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Zur Verdeutlichung des erfinderischen Effekts, wurden die Emulsionen der Beispiele 1 bis 8. hergestellt.

Die Zubereitungen konnten nach den dem Fachmann in der Kosmetik bekannten Herstellungsverfahren hergestellt werden. Die Beispiele 1 bis 8 wurden hergestellt, indem Sodium Carboxymethyl Starch oder Tapiokastärke in Butylene Glycol suspendiert wurden. Xanthan Gum wurde in eine auf 75°C temperierte Mischung aus Caprylic/Capric Triglyceride, Glyceryl Stearate Citrate, Behenyl Alcohol und Phenoxyethanol eingearbeitet. Diese hergestellte Mischung wurde zu einer ebenfalls hergestellten Mischung aus Wasser und Glycerin gegeben, welche ebenfalls auf 75°C vortemperiert wurde. Nach Abkühlen auf 30°C wurde die Stärke-Butylene Glycol Suspension zugegeben und homogenisiert.

Die O/W-Emulsionen der Beispiele Bsp.1, Bsp. 6, Bsp.7 und Bsp.8 stellen nicht erfindungsgemäße Vergleichsbeispiele da, während Bsp.2 bis Bsp.5 erfindungsgemäße Emulsionen sind.

| **Inhaltstoffe** | **Bsp.1** | **Bsp.2** | **Bsp.3** | **Bsp.4** |
|---|---|---|---|---|
| Aqua | ad 100 | ad 100 | ad 100 | ad 100 |
| Glycerin | 8 | 7,96 | 7,92 | 7,84 |
| Caprylic/Capric Triglyceride | 10 | 9,95 | 9,9 | 9,8 |
| Glyceryl Stearate Citrate | 2 | 1,99 | 1,98 | 1,96 |
| Behenyl Alcohol | 3 | 2,985 | 2,97 | 2,94 |
| Phenoxyethanol | 0,8 | 0,796 | 0,792 | 0,784 |
| Xanthan Gum | 0,3 | 0,2985 | 0,297 | 0,294 |
| Sodium Carboxymethyl Starch | | 0,5 | 1 | 2 |
| Tapioca Starch | | | | |
| Butylene Glycol | 5 | 4,975 | 4,95 | 4,9 |
| | | | | |
| | | | | |
| Verhältnis Wasser: Natriumcarboxymethylstärke | - | 141:1 | 70,19:1 | 34,74:1 |

| **Inhaltstoffe** | **Bsp.5** | **Bsp.6** | **Bsp.7** | **Bsp.8** |
|---|---|---|---|---|
| Aqua | ad 100 | ad 100 | ad 100 | ad 100 |
| Glycerin | 7,76 | 7,68 | 7,6 | 7,6 |
| Caprylic/Capric Triglyceride | 9,7 | 9,6 | 9,5 | 9,5 |
| Glyceryl Stearate Citrate | 1,94 | 1,92 | 1,9 | 1,9 |
| Behenyl Alcohol | 2,91 | 2,88 | 2,85 | 2,85 |
| Phenoxyethanol | 0,776 | 0,768 | 0,76 | 0,76 |
| Xanthan Gum | 0,291 | 0,288 | 0,285 | 0,285 |
| Sodium Carboxymethyl Starch | 3 | 4 | 5 | |
| Tapioca Starch | | | | 5 |
| Butylene Glycol | 4,85 | 4,8 | 4,75 | 4,75 |
| | | | | |
| | | | | |
| Verhältnis Wasser: Natriumcarboxymethylstärke bzw. Tapioca starch | 22,92:1 | 17,02:1 | 13,47:1 | 13,47:1 |

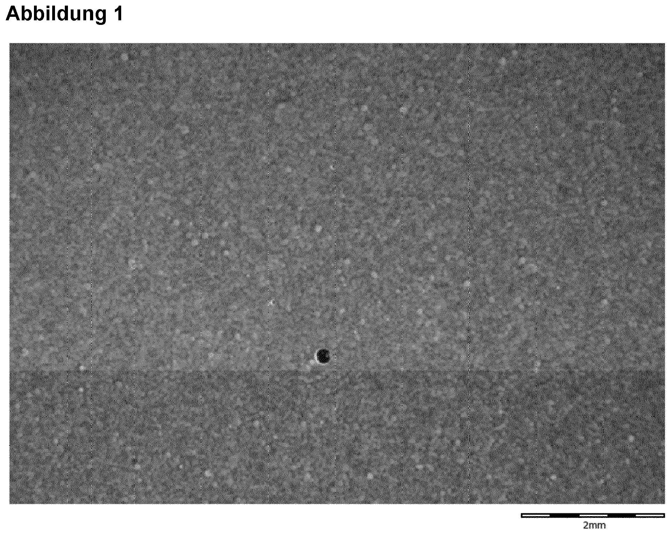

Es wurden Mikroskopaufnahmen der Emulsionen der Beispiele Bsp.1, Bsp.4 und Bsp.8 angefertigt. Diese sind in Abbildung 1 bis 3 dargestellt, wobei immer dieselbe Vergrößerung gewählt wurde.

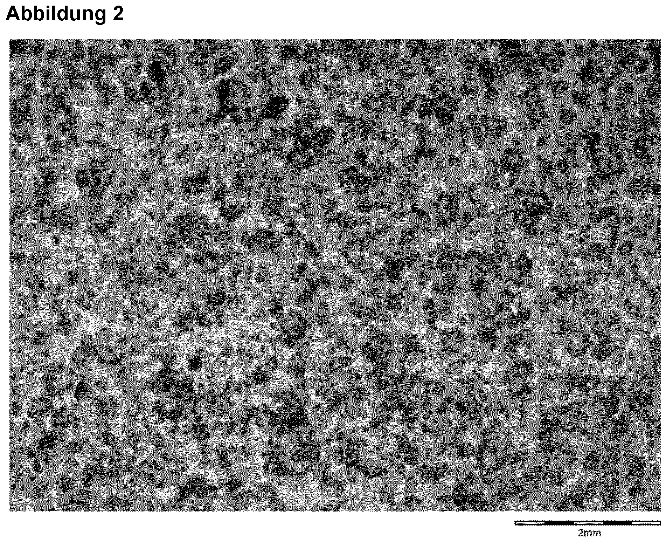

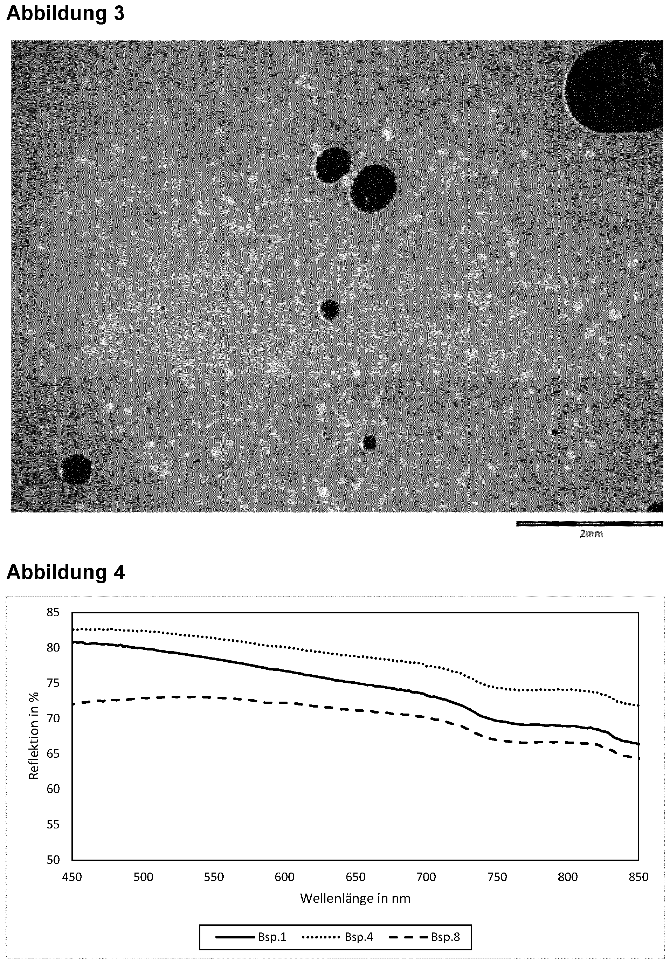

Abbildung 1 zeigt Bsp.1, Abbildung 2 zeigt Bsp.4 und Abbildung 3 zeigt Bsp.8 jeweils 24 Stunden nach Herstellung.

Weiterhin wurde überraschend gefunden, dass die erfindungsgemäßen Beispiele ein überraschend mattes Erscheinungsbild aufgewiesen haben. Dieses zeigt ein Vergleich von Bsp. 1, Bsp.4 und Bsp.8. Dazu wurden Reflektionsmessungen der diffusen Streuung mit einem UV/Vis Spektrometer mit einer Glanzfalle für diese Beispielrezepturen ermittelt.

Das Vergleichsspektrum ist in Abbildung 4 dargestellt. Es ergibt sich, dass der Anteil der diffusen Streuung des Lichts über das gesamte Spektrum am deutlich größer ist für Bsp.4, als Bsp.1 und Bsp.8. Als Folge hat Bsp.4 ein deutlich matteres Erscheinungsbild als Bsp.1 und Bsp.8.

Nachfolgend sind weitere erfindungsgemäße Beispiel aufgeführt:

| **Inhaltstoffe** | **Bsp.9** | **Bsp.10** | **Bsp.11** | **Bsp.12** | **Bsp.13** | **Bsp.14** |
|---|---|---|---|---|---|---|
| Aqua | ad 100 | ad 100 | ad 100 | ad 100 | ad 100 | ad 100 |
| Glycerin | 4,8 | 5,2 | 3,9 | 4,7 | 4,9 | 4 |
| Caprylic/Capric Triglyceride | 7,3 | 6,5 | 7 | 9,3 | 12 | 5 |
| Hydrogenated Coco-Glycerides | 1 | 1,2 | 0,2 | 1,2 | 1,1 | 2 |
| Cetearyl Alcohol | 0,85 | 1 | 0,58 | 2 | 1,55 | 2 |
| Glyceryl Stearate | 1 | 1 | 1 | 1 | 1 | 1 |
| Polyglyceryl-10 Stearate | 1,8 | 1,65 | 1 | 0,5 | 1,2 | 1,2 |
| Fettsäuremischung (1,5 Gew.-% Arachidic Acid; 2,5 Gew.-% Myristic Acid; 0,5 Gew.-% Oleic Acid; 48,5 Gew.-% Palmitic Acid; 47 Gew.-% Stearic Acid) | | | 1 | 2,1 | 0,3 | 0,2 |
| Xanthan Gum | 0,3 | 0,3 | 0,3 | 0,3 | 0,3 | 0,3 |
| Ethanol | 1,92 | 1,92 | 1,92 | 1,92 | 1,92 | 2,3 |
| Phenoxyethanol | 0,6 | 0,8 | 0,5 | 0,8 | 0,7 | 0,8 |
| Butylene Glycol | 5 | 3 | 2 | 5 | 5 | 5 |
| Parfum | 0,3 | 0,5 | 0,3 | 0,3 | 0,3 | 0,3 |
| Tapioca Starch | | | 0,1 | | | |
| Sodium Carboxymethyl Starch | 1,7 | 1,55 | 2 | 1,45 | 2,8 | 2,4 |

| **Inhaltstoffe** | **Bsp.15** | **Bsp.16** | **Bsp.17** | **Bsp.18** | **Bsp.19** |
|---|---|---|---|---|---|
| Aqua | ad 100 | ad 100 | ad 100 | ad 100 | ad 100 |
| Glycerin | 7,0 | 5,0 | 4,0 | 5,0 | 8,0 |
| Aqua + Trisodium EDTA | 0,03 | 0,03 | 0,03 | 0,03 | 0,03 |
| Caprylic/Capric Triglyceride | 6,0 | 6,0 | 6,0 | 4,0 | 6,0 |
| Behenyl Alcohol | 2,0 | 2,0 | 2,0 | 2,0 | 2,0 |
| Glyceryl Stearate | 1,0 | 1,0 | 1,0 | 1,0 | 1,0 |
| Fettsäuremischung (1,5 Gew.-% Arachidic Acid; 2,5 Gew.-% Myristic Acid; 0,5 Gew.-% Oleic Acid; 48,5 Gew.-% Palmitic Acid; 47 Gew.-% Stearic Acid) | 2,5 | 2,5 | 2,5 | 2,5 | 2,5 |
| Hydrogenated Coco-Glycerides | 1,0 | 1,0 | 1,0 | 1,0 | 1,0 |
| Dimethicone | 3,0 | 3,0 | 3,0 | 2,0 | 3,0 |
| Butyrospermum Parkii Butter | 1,0 | 1,0 | 1,0 | 1,0 | 1,0 |
| PVP | 0,9 | 0,9 | 0,9 | 0,9 | 0,9 |
| Xanthan Gum | 0,2 | 0,2 | 0,2 | 0,2 | 0,2 |
| Phenoxyethanol | | 0,5 | 1,0 | 0,8 | 0,8 |
| Ethanol | 5,0 | 4,0 | 2,0 | 2,0 | 2,0 |
| Benzethonium chlorid | 0,05 | | | | |
| Octandiol | 0,2 | 0,3 | | | |
| Butylene Glycol | 4,0 | 5,0 | 5,0 | 5,0 | 4,0 |
| Parfum | 0,3 | 0,3 | 0,3 | 0,3 | 0,3 |
| Sodium Carboxymethyl Starch | 1,5 | 1,75 | 1,8 | 2,5 | 1,5 |
| Butylmethoxydibenzoylmethan | | | | 0,5 | |
| Bis-Ethylhexyloxyphenolmethoxyphenyltriazine | | | | 0,5 | |
| 2-Ethylhexyl-2-cyano-3-diphenylacrylat (Octocrylen) | | | | 2 | |
| Ethylhexylsalicylate | | | | 3 | |
| CI 77492 + CI 77491 + CI 77499 | 0,3 | | | | |
| CI 77491 + CI 77499 | 0,1 | | | | |
| CI 77491 | 0,07 | | | | |
| CI 77492 | 0,9 | | | | |
| CI 77891 | 6,63 | | | | |
| Silica-treated CI 77491 | | 0,17 | | | |
| Silica-treated CI 77499 | | 0,07 | | | |
| Silica-treated CI 338073 | | 0,1 | | | |
| Silica-treated CI 77891 | | 6,76 | | | |
| Alginate-treated Red Iron Oxide | | | 0,17 | | |
| Alginate-treated Black Iron Oxide | | | 0,07 | | |
| Alginate-treated Yellow Iron Oxide | | | 0,1 | | |
| Alginate-treated Pigmentary Titanium Dioxide | | | 6,76 | | |
| Hydrogenated-Lecithin-treated CI 77491 | | | | | 0,17 |
| Hydrogenated-Lecithin-treated CI 77499 | | | | | 0,07 |
| Hydrogenated-Lecithin-treated CI 77492 | | | | | 0,1 |
| Hydrogenated-Lecithin-treated CI 77891 | | | | | 6,76 |

## Patentansprüche

1. Kosmetische Emulsion enthaltend
a) Natriumcarboxymethylstärke und
b) Wasser;
**dadurch gekennzeichnet, dass** das Gewichtsverhältnis von Wasser zu Natriumcarboxymethylstärke weniger als 160:1 und mehr als 20:1 beträgt.

2. Kosmetische Emulsion nach Anspruch 1 **dadurch gekennzeichnet, dass** das Gewichtsverhältnis von Wasser zu Natriumcarboxymethylstärke weniger als 100:1, bevorzugt weniger als 80:1 beträgt und insbesondere bevorzugt weniger als 60:1.

3. Kosmetische Emulsion nach einem der vorhergehenden Ansprüche **dadurch gekennzeichnet, dass** das Gewichtsverhältnis von Wasser zu Natriumcarboxymethylstärke mehr als 30:1 beträgt.

4. Kosmetische Emulsion nach Anspruch 1 **dadurch gekennzeichnet, dass** das Gewichtsverhältniss von Wasser zu Natriumcarboxymethylstärke im Bereich von 80:1 bis 20:1 und insbesondere bevorzugt von 60:1 bis 30:1 liegt.

5. Kosmetische Emulsion nach einem der vorhergehenden Ansprüche **dadurch gekennzeichnet, dass** der Anteil von Wasser in der Emulsion von 55 bis 90 Gew.-%, bevorzugt von 60 bis 80 Gew.-% und insbesondere bevorzugt von 65 bis 75 Gew.-%, bezogen auf das Gesamtgewicht der Emulsion, beträgt.

6. Kosmetische Emulsion nach einem der vorhergehenden Ansprüche **dadurch gekennzeichnet, dass** keine Acrylat-basierten Polymere enthalten sind.

7. Kosmetische Emulsion nach einem der vorhergehenden Ansprüche **dadurch gekennzeichnet, dass** zusätzlich mindestens ein weiteres Polymer auf Basis von Polysacchariden mit Ausnahme von Polysacchariden, welche mit Acrylsäure oder Methacrylsäure modifiziert wurden, enthalten ist.

8. Kosmetische Emulsion nach einem der vorhergehenden Ansprüche **dadurch gekennzeichnet, dass** Xanthan Gum, Sclerotium Gum; Locust Bean Gum und / oder Gellan Gum in einem Anteil von 0,05 bis 0,5 Gew.-% in der Emulsion enthalten ist, wobei sich die Angabe auf das Gesamtgewicht der Emulsion bezieht.

9. Kosmetische Emulsion nach einem der vorhergehenden Ansprüche **dadurch gekennzeichnet, dass** der Anteil der Ölphase von 3 bis 20 Gew.-%, bevorzugt von 5 bis 18 Gew.-%, weiterhin bevorzugt von 6 bis 15 Gew.-% und insbesondere bevorzugt von 7 bis 12 Gew.-% beträgt, wobei Emulgatoren und Tenside definitionsgemäß nicht zur Ölphase zählen und sich die Angaben auf das Gesamtgewicht der Emulsion beziehen.

10. Kosmetische Emulsion nach einem der vorhergehenden Ansprüche **dadurch gekennzeichnet, dass** als Emulgatoren Glyceryl Stearate, Glyceryl Stearate Citrate, Polyglyceryl-10 Stearate und/oder Fettsäuren aufweisend 14 bis 22 Kohlenstoffe und/oder deren Salze enthalten sind.

11. Kosmetische Emulsion nach einem der vorhergehenden Ansprüche **dadurch gekennzeichnet, dass** der Gesamtanteil von Emulgatoren vorteilhaft von 0,1 bis 10 Gew.-%, bevorzugt von 0,5 bis 8 Gew.-% und insbesondere bevorzugt von 1 bis 6 Gew.-% bezogen auf das Gesamtgewicht der Emulsion beträgt.

12. Kosmetische Emulsion nach einem der vorhergehenden Ansprüche **dadurch gekennzeichnet, dass** Stearylalkohol, Cetylalkohol und/oder Behenylalkohol in einem Anteil von 0,5 bis 4 Gew.-% bezogen auf das Gesamtgewicht der Emulsion enthalten sind.

13. Kosmetische Emulsion nach einem der vorhergehenden Ansprüche **dadurch gekennzeichnet, dass** 0,1 bis 12 Gew.-%, bevorzugt 3 bis 10 Gew.-% und insbesondere bevorzugt 5 bis 9 Gew.-% anorganische Pigmente enthalten sind, wobei sich die Angabe auf das Gesamtgewicht der Emulsion bezieht.

14. Kosmetische Emulsion nach Anspruch 14 **dadurch gekennzeichnet, dass** der Anteil der anorganischen Pigmente von 5 bis 10 Gew.-% bezogen auf das Gesamtgewicht der Emulsion beträgt, wobei der Anteil der gecoateten anorganischen Pigmente mindestens 60 Gew.-%, bevorzugt mindestens 90 Gew.-% bezogen auf das Gesamtgewicht aller enthaltenen anorganischen Pigment beträgt.

15. Verwendung von Natriumcarboxymethylstärke zur Ausbildung von Mousse- und/oder Schaumstrukturen in einer kosmetischen wasserhaltigen Emulsion, **dadurch gekennzeichnet, dass** das Gewichtsverhältnis von Wasser zu Natriumcarboxymethylstärke weniger als 160:1 und mehr als 20:1 beträgt.

## Claims

1. Cosmetic emulsion comprising
a) sodium carboxymethyl starch and
b) water;
**characterized in that** the weight ratio of water to sodium carboxymethyl starch is less than 160:1 and more than 20:1.

2. Cosmetic emulsion according to Claim 1, **characterized in that** the weight ratio of water to sodium carboxymethyl starch is less than 100:1, preferably less than 80:1, and particularly preferably less than 60:1.

3. Cosmetic emulsion according to either of the preceding claims, **characterized in that** the weight ratio of water to sodium carboxymethyl starch is more than 30:1.

4. Cosmetic emulsion according to Claim 1, **characterized in that** the weight ratio of water to sodium carboxymethyl starch is within a range from 80:1 to 20:1 and particularly preferably from 60:1 to 30:1.

5. Cosmetic emulsion according to any of the preceding claims, **characterized in that** the proportion of water in the emulsion is from 55% to 90% by weight, preferably from 60% to 80% by weight, and particularly preferably from 65% to 75% by weight, based on the total weight of the emulsion.

6. Cosmetic emulsion according to any of the preceding claims, **characterized in that** it contains no acrylatebased polymers.

7. Cosmetic emulsion according to any of the preceding claims, **characterized in that** it additionally comprises at least one further polysaccharide-based polymer other than polysaccharides modified with acrylic acid or methacrylic acid.

8. Cosmetic emulsion according to any of the preceding claims, **characterized in that** xanthan gum, sclerotium gum, locust bean gum, and/or gellan gum are present in the emulsion in a proportion of 0.05% to 0.5% by weight based on the total weight of the emulsion.

9. Cosmetic emulsion according to any of the preceding claims, **characterized in that** the proportion of the oil phase is from 3% to 20% by weight, preferably from 5% to 18% by weight, more preferably from 6% to 15% by weight, and particularly preferably from 7% to 12% by weight, with emulsifiers and surfactants by definition not counted toward the oil phase and based on the total weight of the emulsion.

10. Cosmetic emulsion according to any of the preceding claims, **characterized in that** it comprises as emulsifiers glyceryl stearate, glyceryl stearate citrate, polyglyceryl-10 stearate, and/or fatty acids containing 14 to 22 carbon atoms and/or salts thereof.

11. Cosmetic emulsion according to any of the preceding claims, **characterized in that** the total proportion of emulsifiers is advantageously from 0.1% to 10% by weight, preferably from 0.5% to 8% by weight, and particularly preferably from 1% to 6% by weight, based on the total weight of the emulsion.

12. Cosmetic emulsion according to any of the preceding claims, **characterized in that** it comprises stearyl alcohol, cetyl alcohol, and/or behenyl alcohol in a proportion of from 0.5% to 4% by weight based on the total weight of the emulsion.

13. Cosmetic emulsion according to any of the preceding claims, **characterized in that** it comprises 0.1% to 12% by weight, preferably 3% to 10% by weight, and particularly preferably 5% to 9% by weight, of inorganic pigments, based on the total weight of the emulsion.

14. Cosmetic emulsion according to Claim 14, **characterized in that** the proportion of inorganic pigments is from 5% to 10% by weight based on the total weight of the emulsion, the proportion of coated inorganic pigments being at least 60% by weight, preferably at least 90% by weight, based on the total weight of all inorganic pigments present.

15. Use of sodium carboxymethyl starch for the formation of mousse structures and/or foam structures in a cosmetic water-containing emulsion, **characterized in that** the weight ratio of water to sodium carboxymethyl starch is less than 160:1 and more than 20:1.

## Revendications

1. Émulsion cosmétique contenant
a) du carboxyméthylamidon sodique et
b) de l'eau ;
**caractérisée en ce que** le rapport en poids de l'eau au carboxyméthylamidon sodique est inférieur à 160:1 et supérieur à 20:1.

2. Émulsion cosmétique selon la revendication 1, **caractérisée en ce que** le rapport en poids de l'eau au carboxyméthylamidon sodique est inférieur à 100:1, de préférence inférieur à 80:1, et tout particulièrement inférieur à 60:1.

3. Émulsion cosmétique selon l'une des revendications précédentes, **caractérisée en ce que** le rapport en poids de l'eau au carboxyméthylamidon sodique est supérieur à 30:1.

4. Émulsion cosmétique selon la revendication 1, **caractérisée en ce que** le rapport en poids de l'eau au carboxyméthylamidon sodique est compris dans la plage de 80:1 à 20:1 et tout particulièrement de 60:1 à 30:1.

5. Émulsion cosmétique selon l'une des revendications précédentes, **caractérisée en ce que** la proportion de l'eau dans l'émulsion est de 55 à 90 % en poids, de préférence de 60 à 80 % en poids et tout particulièrement de 65 à 75 % en poids, par rapport au poids total de l'émulsion.

6. Émulsion cosmétique selon l'une des revendications précédentes, **caractérisée en ce qu'**elle ne contient aucun polymère à base d'acrylate.

7. Émulsion cosmétique selon l'une des revendications précédentes, **caractérisée en ce qu'**elle contient en outre au moins un autre polymère à base de polysaccharides à l'exception des polysaccharides qui ont été modifiés par de l'acide acrylique ou de l'acide méthacrylique.

8. Émulsion cosmétique selon l'une des revendications précédentes, **caractérisée en ce qu'**elle contient de la gomme xanthane, de la gomme de sclérotium, de la gomme de caroube et/ou de la gomme gellane, selon une proportion de 0,05 à 0,5 % en poids dans l'émulsion, par rapport au poids total de l'émulsion.

9. Émulsion cosmétique selon l'une des revendications précédentes, **caractérisée en ce que** la proportion de la phase huileuse est de 3 à 20 % en poids, de préférence de 5 à 18 % en poids, plus préférentiellement de 6 à 15 % en poids et tout particulièrement de 7 à 12 % en poids, les émulsifiants et les tensioactifs, par définition, n'appartenant pas à la phase huileuse, par rapport au poids total de l'émulsion.

10. Émulsion cosmétique selon l'une des revendications précédentes, **caractérisée en ce qu'**elle contient en tant qu'émulsifiants du stéarate de glycéryle, du stéarate et citrate de glycéryle, du stéarate de polyglycéryle-10 et/ou des acides gras ayant 14 à 22 atomes de carbone et/ou des sels de ceux-ci.

11. Émulsion cosmétique selon l'une des revendications précédentes, **caractérisée en ce que** la proportion totale des émulsifiants est avantageusement de 0,1 à 10 % en poids, de préférence de 0,5 à 8 % en poids et tout particulièrement de 1 à 6 % en poids par rapport au poids total de l'émulsion.

12. Émulsion cosmétique selon l'une des revendications précédentes, **caractérisée en ce qu'**elle contient de l'alcool stéarylique, de l'alcool cétylique et/ou de l'alcool béhénylique, selon une proportion de 0,5 à 4 % en poids par rapport au poids total de l'émulsion.

13. Émulsion cosmétique selon l'une des revendications précédentes, **caractérisée en ce qu'**elle contient 0,1 à 12 % en poids, de préférence 3 à 10 % en poids et tout particulièrement 5 à 9 % en poids de pigments inorganiques, par rapport au poids total de l'émulsion.

14. Émulsion cosmétique selon la revendication 14, **caractérisée en ce que** la proportion des pigments inorganiques est de 5 à 10 % en poids par rapport au poids total de l'émulsion, la proportion des pigments inorganiques revêtus étant d'au moins 60 % en poids, de préférence d'au moins 90 % en poids par rapport au poids total de la totalité des pigments inorganiques présents.

15. Utilisation de carboxyméthylamidon sodique pour réaliser des structures de mousse et/ou expansées dans une émulsion cosmétique aqueuse, **caractérisée en ce que** le rapport en poids de l'eau au carboxyméthylamidon sodique est inférieur à 160:1 et supérieur à 20:1.
